# EUROPÄISCHE PATENTSCHRIFT

(11) **EP 4 473 843 B1**
(45) Veröffentlichungstag und Bekanntmachung des Hinweises auf die Patenterteilung: **08.10.2025**
(21) Anmeldenummer: 24155473.2
(22) Anmeldetag: 02.02.2024
(51) Int. Cl.: A23B 2/20

(54) **SYSTEM UND VERFAHREN ZUM ERWÄRMEN UND/ODER ABKÜHLEN, INSBESONDERE PASTEURISIEREN, VON GEFÜLLTEN UND GESCHLOSSENEN BEHÄLTERN**
SYSTEM AND METHOD FOR HEATING AND/OR COOLING FILLED CONTAINERS, IN PARTICULAR PASTEURIZING, AND CLOSED CONTAINERS
SYSTÈME ET PROCÉDÉ DE CHAUFFAGE ET/OU DE REFROIDISSEMENT, EN PARTICULIER PASTEURISATION, DE RÉCIPIENTS REMPLIS ET FERMÉS

(30) Priorität: 06.06.2023 DE 102023114816
(43) Veröffentlichungstag der Anmeldung: 11.12.2024
(73) Patentinhaber: KRONES Aktiengesellschaft, 93073 Neutraubling (DE)
(72) Erfinder: Kaatz, Stefan, 93073 Neutraubling (DE); Schmuck, Jesse, 93073 Neutraubling (DE)
(74) Vertreter: Grünecker Patent- und Rechtsanwälte PartG mbB

(56) Entgegenhaltungen:
- EP-B1- 2 702 879
- EP-B1- 2 833 742
- US-A- 5 772 958

## Beschreibung

Die Erfindung betrifft ein System und ein Verfahren zum Erwärmen und/oder Abkühlen, insbesondere Pasteurisieren, von mit einem insbesondere flüssigen Füllgut gefüllten und geschlossenen Behältern.

Vorrichtungen zum Erwärmen und/oder Abkühlen von gefüllten und geschlossenen Behältern finden regelmäßig Anwendung in automatisierten Anlagen der Konsumgüter- und/oder Lebensmittelindustrie z.B. in Form eines Pasteurs.

Durch die Integration des Pasteurs in eine automatisierte Anlage, die mehrere aufeinanderfolgende Maschinen umfasst, besteht ein Bedarf der Anpassung der Systemparameter der einzelnen Maschinen, insbesondere der Transportgeschwindigkeiten der einzelnen Maschinen, um einen reibungslosen Gesamtablauf zu gewährleisten.

EP 2 833 742 B1 veröffentlicht eine Vorrichtung bei der eine Transportgeschwindigkeit eines pasteurinternen Transportelementes kontinuierlich steuer- oder regelbar ist. Bei Erhöhung der Transportgeschwindigkeit wird die Anzahl der als Vorwärmzonen dienenden Zonen bei gleichzeitiger Erhöhung der Behandlungstemperatur in diesen Zonen verringert und die Anzahl der als Pasteurisierzonen dienenden Zonen erhöht. Bei einer Reduzierung der Transportgeschwindigkeit wird die Anzahl der als Vorwärmzonen dienenden Zonen bei teilweiser Reduzierung der Behandlungstemperatur erhöht und die Anzahl der als Pasteurisierzonen wirkenden Zonen reduziert.

EP 2 702 879 B1 offenbart ein System und ein Verfahren zur Temperaturregelung in einer Temperaturverarbeitungsmaschine für Nahrungsmittelbehälter.

US 5,772,958 offenbart ein Verfahren und eine Vorrichtung zum Pasteurisieren einer kontinuierlichen Produktlinie.

Alternativ ist aus dem Stand der Technik ein Ansatz bekannt, bei dem im Fall einer eingeschränkten Verfügbarkeit von Produkten am Einlauf der Pasteur stehen bleibt und wieder mit voller Leistung weiterfährt, sobald genügend Produkte vorhanden sind. Entsprechend bleibt der Pasteur bei Stau am Auslauf stehen und fährt nach Auflösung des Staus mit voller Leistung weiter.

Bei diesem Ansatz muss die Prozessregelung bei jedem Start davon ausgehen, dass stets die volle Transportleistung erbracht wird. Um eine ausreichende Produktbehandlung zu gewährleisten wird die Temperatur des Behandlungsmediums entsprechend stark angehoben. Kommt es allerdings in Folge von Stau und/oder eingeschränkter Verfügbarkeit von Produkten am Einlauf und/oder Auslauf zu zeitweisem Stillstand des Pasteurs, so kann dies zu einer Überpasteurisierung der Produkte führen.

Angesichts dessen besteht die der Erfindung zugrundeliegende Aufgabe darin, den Energieverbrauch bei der Wärmebehandlung von gefüllten und geschlossenen Behältern zu reduzieren bei guten Pasteurisierungsergebnissen.

Diese Aufgabe wird durch ein System gemäß Anspruch 1 gelöst.

Die Erfindung stellt ein System zum Erwärmen und/oder Abkühlen, insbesondere Pasteurisieren, von mit einem insbesondere flüssigen Füllgut gefüllten und geschlossenen Behältern bereit. Das System umfasst eine Transportvorrichtung ausgebildet zum Transportieren der Behälter in einer Transportrichtung, eine Vorrichtung ausgebildet zum Erwärmen und/oder Abkühlen der Behälter gemäß einem Temperaturprofil entlang der Transportrichtung, und eine Steuerungsvorrichtung ausgebildet zum Steuern einer Transportgeschwindigkeit der Transportvorrichtung und des Temperaturprofils. Dabei ist die Steuerungsvorrichtung ausgebildet, die Transportgeschwindigkeit im zeitlichen Wechsel zwischen Stopp und einer Nenn-Transportgeschwindigkeit zu steuern und das Temperaturprofil abhängig von einem prognostizierten Systemparameter zu steuern.

Das System kann insbesondere ein Tunnelpasteur sein.

Dadurch, dass die Transportgeschwindigkeit zwischen Stopp und einer Nenn-Transportgeschwindigkeit wechselt, würde eine Anpassung des Temperaturprofils nur auf Grund der derzeitigen Transportgeschwindigkeit zu einem recht schnellen Wechsel der Temperaturen in dem System führen. Da die Temperaturen in dem System aber im Vergleich zu der Zeit innerhalb der sich die Transportgeschwindigkeit (auch mehrmals) ändert, recht träge sind, scheint es besser zu sein, das Temperaturprofil anhand von einem prognostizierten Systemparameter zu steuern, als an Hand von (nur) einem aktuellen Systemparameter.

Der Wechsel zwischen Stopp und einer Nenn-Transportgeschwindigkeit findet beispielsweise so statt, dass für die Transportgeschwindigkeit nur diese Werte durch die Steuerung vorgegeben werden und keine Zwischenwerte, wie etwa eine Transportgeschwindigkeit von 30% oder 50% oder 70% der Nenn-Transportgeschwindigkeit.

Für einen (im Vergleich zu dem trägen Temperaturregelsystem des Systems) schnellen Wechsel zwischen Stopp und der Nenn-Transportgeschwindigkeit bedingt z.B. durch eine Auslastung des Systems mir weniger als seiner Nennleistung, ist eine Steuerung des Temperaturprofils mit einem prognostizierten Systemparameter besser als eine Steuerung auf Grund der sich vergleichsweise schnell ändernden Ist-Transportgeschwindigkeit.

Unter Pasteurisieren kann das kurzzeitige Erwärmen auf Temperaturen von 60 bis 90°C zu verstehen sein.

In manchen Fällen ist das flüssige Füllgut ein Lebensmittel, insbesondere ein Saft oder eine Milch.

Mit Behältern können Lebensmittelverpackungen wie etwa Flaschen, Dosen oder Kanister gemeint sein. Die Behälter können mittels Deckeln und/oder Verschlüssen und/oder Folien verschlossen sein.

Die Transportvorrichtung kann ein Förderband sein oder umfassen. Die Transportrichtung ist die Richtung, in die sich die Transportvorrichtung bewegt.

Die Vorrichtung ausgebildet zum Erwärmen und/oder Abkühlen der Behälter ist beispielsweise vorgesehen, die Behälter mit einem flüssigen Behandlungsmedium, insbesondere Wasser, einer steuerbaren und damit einstellbaren Temperatur zu beaufschlagen. Die gewünschte Temperatur des Behandlungsmediums ist durch das Temperaturprofil bestimmt.

Die Steuerungsvorrichtung kann Steuersignale basierend auf Daten erzeugen und insbesondere die Steuersignale an Regelungsvorrichtungen und/oder Komponenten senden.

Der Systemparameter kann beispielsweise die Transportgeschwindigkeit, eine Anzahl der in einer Zeiteinheit einlaufenden Behälter, eine Anzahl der in einer Zeiteinheit abgebbaren Behälter, die Länge des vollen Teils einer Staustrecke, die Anzahl der (gestauten) Behälter in einer Staustrecke, eine Aufnahme- und/oder Ausgabekapazität, und/oder eine Systemauslastung in Prozent, jeweils des Systems, umfassen.

Durch die Steuerung des Temperaturprofils abhängig von dem prognostizierten Systemparameter, der Ausdruck eines Durchsatzes des Systems an Behältern je Zeiteinheit in der Zukunft sein kann, kann auf eine voraussichtlich benötigte Energiemenge zur Wärmebehandlung der Behälter und entsprechend das Temperaturprofil rückgeschlossen werden. Dadurch wird die zugrundeliegende Aufgabe gelöst, die Kosten und den Energieverbrauch, sowie den negativen Einfluss einer Überpasteurisierung auf einfache Art und Weise zu reduzieren.

Hier und im Folgenden kann die Aufnahmekapazität umfassen, wieviele Behälter aufgenommen werden können. Entsprechend kann hier und im Folgenden mit Ausgabekapazität gemeint sein, wieviele Behälter ausgegeben werden können. Die Aufnahme- und/oder Ausgabekapazität kann eine absolute Größe oder eine relative Größe, insbesondere bezogen auf die gesamte Staukapazität einer Staustrecke, umfassen.

Hier und im Folgenden ist die Systemauslastung ein Anteil eines Ist-Durchsatzes an Behältern je Zeiteinheit an einem maximalen Durchsatz an Behältern je Zeiteinheit. Der maximale Durchsatz kann ein Nenn-Durchsatz sein.

Der Systemparameter kann eine mittlere Transportgeschwindigkeit des Systems sein.

Die Steuerungsvorrichtung ist ausgebildet, die Transportgeschwindigkeit abhängig von einer Anzahl an Behältern in einem Einlauf und/oder Auslauf des Systems in der Vergangenheit und/oder Gegenwart, insbesondere deren zeitlicher Verläufe und/oder einer verfügbaren Kapazität zur Aufnahme von Behältern in dem/einem Einlauf und/oder Auslauf des Systems in der Vergangenheit und/oder Gegenwart, insbesondere deren zeitlicher Verläufe, zu steuern.

Durch eine so erfolgende Steuerung der Transportgeschwindigkeit wird ein Stau oder Mangel am Einlauf und/oder Auslauf und somit ein kritischer Betriebszustand und/oder Betriebszustand mit erhöhtem Energieverbrauch vermieden.

Das System kann eine Erfassungsvorrichtung im Bereich des/eines Einlaufs und/oder Auslaufs des Systems zur Erfassung von Behältern im Bereich des Einlaufs und/oder Auslaufs des Systems umfassen. Dabei kann die Erfassungsvorrichtung einen Stauschalter und/oder eine Bilderfassungsvorrichtung umfassen, wobei die Steuerungsvorrichtung insbesondere ausgebildet sein kann, die Transportgeschwindigkeit basierend auf Daten der Erfassungsvorrichtung zu steuern.

Durch die Erfassungsvorrichtung im Bereich des Einlaufs und/oder Auslaufs des Systems werden Daten über das System auf einfache Art und Weise erfasst, die direkten Einfluss auf den Betriebszustand des Systems haben. Durch die Erfassungsvorrichtung ist es möglich, die Behälter im Bereich des Einlaufs und/oder Auslauf des Systems zu erfassen und die Transportgeschwindigkeit abhängig von dem tatsächlichen Vorhandensein von Behältern im Bereich des Einlaufs und/oder Auslaufs, insbesondere in Fällen des Mangels und/oder Staus im Einlauf und/oder Auslauf zu steuern. Dadurch werden kritische Betriebszustände und/oder Betriebszustände mit erhöhtem Energieverbrauch auf einfache Art und Weise vermieden.

In manchen Fällen umfasst der Stauschalter einen beweglichen Bügel und einen Sensor, wobei der Bügel bei steigendem Staudruck bewegt wird und die geänderte Position des Bügels von dem Sensor erfasst wird.

Die Bilderfassungsvorrichtung kann z.B. eine Kamera sein.

Mit Daten der Erfassungsvorrichtung können ein Signal des Sensors des Stauschalters und/oder ein Bild der Bilderfassungsvorrichtung gemeint sein.

Die Steuerungsvorrichtung kann ausgebildet sein, den Systemparameter basierend auf einer Transportgeschwindigkeit, einer Anzahl der in einer Zeiteinheit einlaufenden Behälter, einer Anzahl der in einer Zeiteinheit abgebbaren Behälter, der/einer Länge des vollen Teils einer Staustrecke, einer Anzahl von (gestauten) Behältern in einer Staustrecke, einer Aufnahme- und/oder Ausgabekapazität, und/oder einer Systemauslastung in Prozent jeweils des Systems, und/oder eines oder mehrerer dem System vor- und/oder nachgeordneten weiteren Systemen in der Vergangenheit und/oder Gegenwart zu prognostizieren.

Durch die Abhängigkeit des prognostizierten Systemparameters des Systems von einem oder mehreren Systemparametern des Systems und/oder eines oder mehrerer dem System vor- und/oder nachgeordneten weiteren Systeme in der Vergangenheit und/oder Gegenwart kann der Systemparameter des Systems zuverlässig prognostiziert werden. Ein Vorteil dieser Art der Prognose ist es, dass keine anderen Daten als bekannte, inhärente Systemparameter benötigt werden.

Die Vorrichtung kann entlang der Transportrichtung der Behälter mehrere Temperaturzonen aufweisen. Dabei kann das Temperaturprofil steuerbare Temperaturen der Temperaturzonen angeben. Eine Steuerung des Temperaturprofils kann eine Steuerung der Temperaturen der Temperaturzonen umfassen, wobei jede Temperaturzone eine für sich genommen einheitliche Temperatur aufweisen kann.

Durch das Vorhandensein mehrerer Temperaturzonen der Vorrichtung erfolgt eine bedarfsgerechte Pasteurisierung der Behälter.

In dem System können in mehreren als Aufwärmzonen bezeichneten Temperaturzonen, die in der Transportrichtung aufeinanderfolgen können, die Temperaturen von Temperaturzone zu Temperaturzone ansteigen oder gleichbleiben. Die Aufwärmzonen dienen dazu, das Füllgut der Behälter aufzuwärmen. Zusätzlich kann in wenigstens einer oder mehrerer in der Transportrichtung den Aufwärmzonen nachfolgenden als Pasteurisierungszonen bezeichneten Temperaturzonen die Temperatur einer Maximaltemperatur (des Temperaturprofils) entsprechen. Hier findet eine Pasteurisierung des Füllguts statt. Zusätzlich können in mehreren in der Transportrichtung den Pasteurisierungszonen nachfolgenden als Abkühlzonen bezeichneten Temperaturzonen die Temperaturen von Temperaturzone zu Temperaturzone abnehmen oder gleichbleiben. Die Abkühlzonen dienen dazu, das Füllgut der Behälter abzukühlen und so den Pasteurisierungsprozess in einem Behälter zu beenden.

Durch die Einteilung der Temperaturzonen nach Funktionen wird die Kontrolle der Wärmebehandlung der Behälter verbessert.

In den mehreren Aufwärmzonen können die Temperaturen von Temperaturzone zu Temperaturzone schrittweise und/oder graduell ansteigen. Die Temperaturen der Aufwärmzonen können niedriger als die Maximaltemperatur (des Temperaturprofils) sein.

In den mehreren Abkühlzonen können die Temperaturen von Temperaturzone zu Temperaturzone schrittweise und/oder graduell abnehmen. Die Temperaturen der Abkühlzonen können niedriger als die Maximaltemperatur sein.

Die Steuerungsvorrichtung kann ausgebildet sein, eine Anzahl der Pasteurisierungszonen zu erhöhen. Dies geschieht falls a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, zunimmt und/oder b) eine prognostizierte Anzahl der in einer Zeiteinheit abgebbaren Behälter, und/oder eine prognostizierte Aufnahmekapazität, jeweils des Systems, abnimmt.

Alternativ oder zusätzlich kann die Steuerungsvorrichtung ausgebildet sein, eine/die Anzahl der Pasteurisierungszonen zu reduzieren, falls a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, abnimmt und/oder eine prognostizierte Anzahl der in einer Zeiteinheit abgebbaren Behälter, und/oder eine prognostizierte Aufnahmekapazität, jeweils des Systems, zunimmt.

Durch die Anpassung der Anzahl der Pasteurisierungszonen an prognostizierte Systemparameter wird die zu erzielende Wärmebehandlung der Behälter bedarfsgerecht kontrolliert.

Die Steuerungsvorrichtung kann ausgebildet sein, die Transportgeschwindigkeit, die Anzahl der in einer Zeiteinheit einlaufenden Behälter, die Ausgabekapazität, die Anzahl der in einer Zeiteinheit abgebbaren Behälter, die Aufnahmekapazität, und/oder die Systemauslastung in Prozent basierend auf der/einer Transportgeschwindigkeit, der/einer Anzahl der in einer Zeiteinheit einlaufenden Behälter, der/einer Anzahl der in einer Zeiteinheit abgebbaren Behälter, der/einer Länge des vollen Teils einer Staustrecke, einer Anzahl von (gestauten) Behältern in einer Staustrecke, der/einer Aufnahme- und/oder Ausgabekapazität, und/oder der/einer Systemauslastung in Prozent jeweils des Systems, und/oder eines oder mehrerer dem System vor- und/oder nachgeordneten weiteren Systemen in der Vergangenheit und/oder Gegenwart zu prognostizieren.

Durch die Abhängigkeit des prognostizierten Systemparameters des Systems von einem oder mehreren Systemparametern des Systems und/oder eines oder mehrerer dem System vor- und/oder nachgeordneten weiteren Systeme in der Vergangenheit und/oder Gegenwart wird die Zuverlässigkeit der Prognose des Systemparameters des Systems verbessert.

Die Erfindung stellt weiterhin ein Verfahren zum Erwärmen und/oder Abkühlen, insbesondere Pasteurisieren, von mit einem insbesondere flüssigen Füllgut gefüllten und verschlossenen Behältern mithilfe eines Systems zum Erwärmen und/oder Abkühlen, insbesondere Pasteurisieren, von den verschlossenen Behältern bereit. Das System umfasst eine Transportvorrichtung ausgebildet zum Transportieren der Behälter in einer Transportrichtung, eine Vorrichtung ausgebildet zum Erwärmen und/oder Abkühlen der Behälter gemäß einem Temperaturprofil entlang der Transportrichtung, und eine Steuerungsvorrichtung ausgebildet zum Steuern einer Transportgeschwindigkeit der Transportvorrichtung und des Temperaturprofils. Dabei ist die Steuerungsvorrichtung ausgebildet, die Transportgeschwindigkeit im zeitlichen Wechsel zwischen Stopp und einer Nenn-Transportgeschwindigkeit zu steuern und das Temperaturprofil abhängig von einem prognostizierten Systemparameter zu steuern. Das Verfahren umfasst das Transportieren der Behälter in der Transportrichtung, Erwärmen und/oder Abkühlen der Behälter gemäß dem Temperaturprofil entlang der Transportrichtung, Steuern der Transportgeschwindigkeit der Transportvorrichtung und des Temperaturprofils, wobei die Transportgeschwindigkeit im zeitlichen Verlauf zwischen Stopp und der Nenn-Transportgeschwindigkeit wechselt, Prognostizieren des Systemparameters, und Steuern des Temperaturprofils abhängig von dem prognostizierten Systemparameter.

Ein derartiges Verfahren ermöglicht die Anpassung des Temperaturprofils an einen prognostizierten Systemparameter. Dadurch wird die zugrundeliegende Aufgabe gelöst, die Kosten und den Energieverbrauch, sowie einen negativen Einfluss der Wärmebehandlung auf das Füllgut durch eventuelles Überpasteurisieren zu reduzieren.

Gemäß dem Verfahren kann die Transportgeschwindigkeit abhängig von einer Anzahl an Behältern in einem Einlauf und/oder Auslauf des Systems in der Vergangenheit und/oder Gegenwart, insbesondere deren zeitlicher Verläufe und/oder einer verfügbaren Kapazität zur Aufnahme von Behältern in dem/einem Einlauf und/oder Auslauf des Systems in der Vergangenheit und/oder Gegenwart, insbesondere deren zeitlicher Verläufe, gesteuert werden.

Durch eine so erfolgende Steuerung der Transportgeschwindigkeit wird ein Stau oder Mangel am Einlauf und/oder Auslauf und somit ein kritischer Betriebszustand und/oder Betriebszustand mit erhöhtem Energieverbrauch vermieden.

Das Verfahren kann umfassen Erfassen von Behältern im Bereich des/eines Einlaufs und/oder Auslaufs des Systems, wobei das Erfassen ein Erfassen eines Staus und/oder eines Bildes umfassen kann. Dabei kann insbesondere die Transportgeschwindigkeit basierend auf Daten des Erfassens gesteuert werden.

Durch das Erfassen im Bereich des Einlaufs und/oder Auslaufs des Systems werden Daten über das System auf einfache Art und Weise erfasst, die direkten Einfluss auf den Betriebszustand des Systems haben. Dadurch ist es möglich, die Transportgeschwindigkeit abhängig von dem tatsächlichen Vorhandensein von Behältern im Bereich des Einlaufs und/oder Auslaufs, insbesondere in Fällen des Mangels und/oder Staus im Einlauf und/oder Auslauf zu steuern. Dadurch werden kritische Betriebszustände und/oder Betriebszustände mit erhöhtem Energieverbrauch auf einfache Art und Weise vermieden.

Das Verfahren kann umfassen ein Prognostizieren des Systemparameters basierend auf einer Transportgeschwindigkeit, einer Anzahl der in einer Zeiteinheit einlaufenden Behälter, einer Anzahl der in einer Zeiteinheit abgebbaren Behälter, der/einer Länge des vollen Teils einer Staustrecke, einer Anzahl von (gestauten) Behältern in einer Staustrecke, einer Aufnahme- und/oder Ausgabekapazität, und/oder einer Systemauslastung in Prozent jeweils des Systems, und/oder eines oder mehrerer dem System vor- und/oder nachgeordneten weiteren Systemen in der Vergangenheit und/oder Gegenwart.

Durch die Abhängigkeit des prognostizierten Systemparameters des Systems von einem oder mehreren Systemparametern des Systems und/oder eines oder mehrerer dem System vor- und/oder nachgeordneten weiteren Systeme in der Vergangenheit und/oder Gegenwart kann der Systemparameter des Systems zuverlässig prognostiziert werden. Ein Vorteil dieser Art der Prognose ist es, dass keine anderen Daten als bekannte, inhärente Systemparameter benötigt werden.

Das Verfahren kann umfassen Erhöhen einer Anzahl der Pasteurisierungszonen, falls a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, zunimmt und/oder b) eine prognostizierte Anzahl der in einer Zeiteinheit abgebbaren Behälter, und/oder eine prognostizierte Aufnahmekapazität, jeweils des Systems, abnimmt. Alternativ oder zusätzlich kann das Verfahren umfassen Reduzieren einer/der Anzahl der Pasteurisierungszonen, falls a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, abnimmt und/oder b) eine prognostizierte Anzahl der in einer Zeiteinheit abgebbaren Behälter, und/oder eine prognostizierte Aufnahmekapazität, jeweils des Systems, zunimmt.

Durch die Anpassung der Anzahl der Pasteurisierungszonen an prognostizierte Systemparameter wird die zu erzielende Wärmebehandlung der Behälter bedarfsgerecht kontrolliert.

Das Verfahren kann umfassen Prognostizieren der Transportgeschwindigkeit, der Anzahl der in einer Zeiteinheit einlaufenden Behälter, der Ausgabekapazität, der Anzahl der in einer Zeiteinheit abgebbaren Behälter, der Aufnahmekapazität, und/oder der Systemauslastung in Prozent basierend auf der/einer Transportgeschwindigkeit, der/einer Anzahl der in einer Zeiteinheit einlaufenden Behälter, der/einer Anzahl der in einer Zeiteinheit abgebbaren Behälter, der/einer Länge des vollen Teils einer Staustrecke, einer Anzahl von (gestauten) Behältern in einer Staustrecke, der/einer Aufnahme- und/oder Ausgabekapazität, und/oder der/einer Systemauslastung in Prozent jeweils des Systems, und/oder eines oder mehrerer dem System vor- und/oder nachgeordneten weiteren Systemen in der Vergangenheit und/oder Gegenwart.

Durch die Abhängigkeit des prognostizierten Systemparameters des Systems von einem oder mehreren Systemparametern des Systems und/oder eines oder mehrerer dem System vor- und/oder nachgeordneten weiteren Systeme in der Vergangenheit und/oder Gegenwart wird die Zuverlässigkeit der Prognose des Systemparameters des Systems verbessert.

Die vorliegende Erfindung wird anhand der nachfolgenden beispielhaften Ausführungsformen unter Bezugnahme auf die Figuren näher beleuchtet, ohne die Erfindung auf die speziellen dargestellten Ausführungsformen zu beschränken. Dabei zeigen:
- Fig. 1: schematisch ein System zum Erwärmen und/oder Abkühlen, insbesondere Pasteurisieren, von mit einem insbesondere flüssigen Füllgut gefüllten und geschlossenen Behältern in einer Seitenansicht und entsprechendes Temperaturprofil,
- Fig. 2: ein Blockdiagramm während des Betriebs des Systems der Fig. 1 zum Erwärmen und/oder Abkühlen, insbesondere Pasteurisieren, von mit einem insbesondere flüssigen Füllgut gefüllten und geschlossenen Behältern,
- Fig. 3: schematisch einen beispielhaften zeitlichen Verlauf einer Transportleistung eines Pasteurs, sowie eine prognostizierte Wärmeleistung des Pasteurs abhängig von der Leistung eines vorgeordneten Füllers.

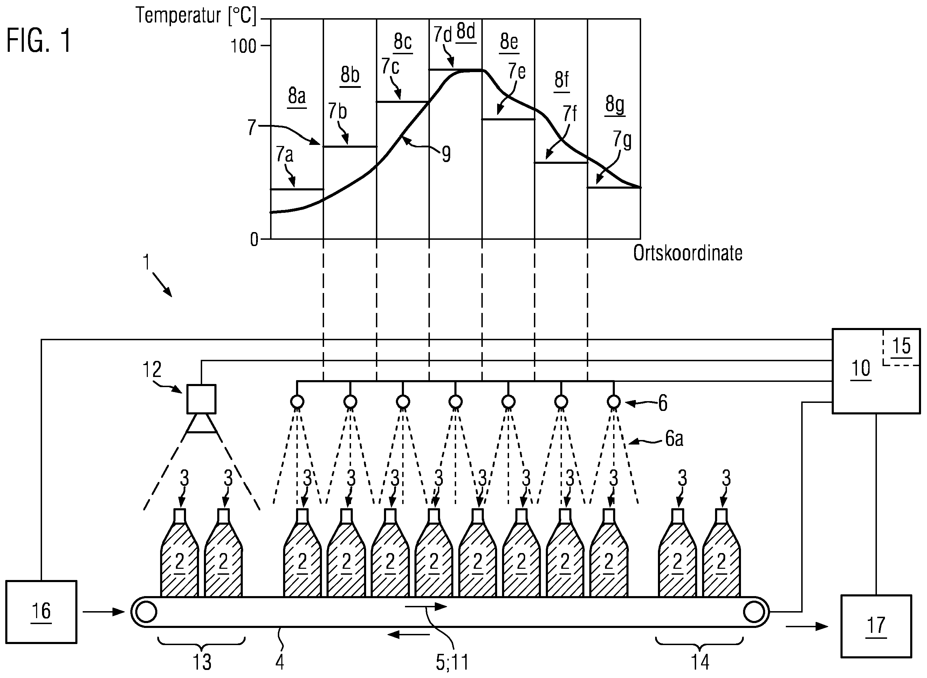

Fig. 1 illustriert den schematischen Aufbau eines Ausführungsbeispiels eines Systems 1 zum Erwärmen und/oder Abkühlen, insbesondere Pasteurisieren, von mit einem insbesondere flüssigen Füllgut gefüllten und geschlossenen Behältern. In dem dargestellten System 1 werden mit einem insbesondere flüssigen Füllgut gefüllte und geschlossene Behälter erwärmt und/oder abgekühlt, insbesondere pasteurisiert.

Ein solches System 1 findet beispielsweise in einer verblockten Anlage der Lebensmittel- und/oder Konsumgüterindustrie im Bereich der Wärmebehandlung von Behältern Anwendung. Vorgeordnet ist beispielsweise eine Einrichtung zum Befüllen und Verschließen der Behälter. Nachgeordnet ist beispielsweise eine Einrichtung zum Etikettieren der Behälter.

Die Fig. 1 zeigt mit einem Füllgut 2, beispielsweise Saft oder Bier, gefüllte Behälter 3, beispielsweise Flaschen. Die Behälter können auch Dosen oder Gläser sein. Die Behälter 3 werden auf einer Transportvorrichtung 4, beispielsweise einem Förderband, in einer Transportrichtung 5 stehend transportiert.

Während des Transports können die Behälter 3 von oberhalb mit einem flüssigen Behandlungsmedium 6a, beispielsweise Wasser, aus Auslässen einer Vorrichtung 6 zum Erwärmen und/oder Abkühlen der Behälter 3 beaufschlagt werden.

Die Temperatur des flüssigen Behandlungsmediums 6a ist beispielsweise zonenweise steuerbar. Ein Temperaturprofil 7 umfasst steuerbare Temperaturen 7a-7g von Temperaturzonen 8a-8g. Beispielsweise umfasst die Vorrichtung 6 sieben Temperaturzonen 8a-8g. Es können auch mehr oder weniger wie etwa 5 bis 12 Temperaturzonen vorgesehen sein. Das flüssige Behandlungsmedium 6a der spezifischen Temperaturzone 8x hat die spezifische Temperatur 7x, wobei x für a, b, c, d, e, f, oder g steht. In Transportrichtung 5 aufeinanderfolgende erste Temperaturzonen 8a-c werden beispielsweise als Aufwärmzonen bezeichnet. Dies können z. B. drei, vier oder fünf oder mehr Temperaturzonen sein. In diesen Temperaturzonen 8a-c werden die Behälter 3 aufgewärmt. Die Temperaturzonen 8a-8c sind beispielsweise durch ein stufenweises Ansteigen der entsprechenden Temperaturen 7a-7c gekennzeichnet. Der Temperaturzone 8c in der Transportrichtung 5 nachfolgend angeordnet ist eine Temperaturzone 8d, die als Pasteurisierungszone bezeichnet wird, deren Temperatur 7d einer Maximaltemperatur des Temperaturprofils entspricht, bzw. deren Temperatur so hoch ist, dass eine Pasteurisierungswirkung eintritt. In der Temperaturzone 8d findet das Pasteurisieren der Behälter 3 statt. Die Temperatur 7d ist die höchste Temperatur unter den Temperaturen 7a-7g. Dies bedeutet, dass sowohl die Temperaturen 7a-7c, als auch die Temperaturen 7e-7g niedriger als die Temperatur 7d sind. Der Temperaturzone 8d in der Transportrichtung 5 nachfolgend angeordnet sind die in Transportrichtung 5 aufeinanderfolgende Temperaturzonen 8e-8g, die als Abkühlzonen bezeichnet werden. In diesen Temperaturzonen 8e-8g können die Behälter abgekühlt werden. Die Temperaturzonen 8e-8g sind beispielsweise durch ein stufenweises Abnehmen der entsprechenden Temperaturen 7e-7g gekennzeichnet.

Wenn die Behälter 3 in Transportrichtung 5 das Temperaturprofil 7 durchlaufen, ändert sich eine Temperatur des Füllguts 2 gemäß einer Temperaturkurve 9. Die Temperaturkurve 9 wurde experimentell durch Messung der Kerntemperatur im Zentrum eines Behälters 3 ermittelt. Die Temperaturkurve 9 steigt beginnend bei einer Anfangstemperatur des Füllguts 2 zu Beginn der Temperaturzone 8a in den Temperaturzonen 8a-8d bis hin zum Erreichen der Maximaltemperatur in der Temperaturzone 8d, bei der die Behälter 3 pasteurisiert werden, an. In den Temperaturzonen 8e-8g nimmt die Temperaturkurve 9 ab.

Die Transportvorrichtung 4 wird durch eine Steuerungsvorrichtung 10 gesteuert. Dies umfasst, dass die Steuerungsvorrichtung 10 beispielsweise die Transportgeschwindigkeit 11 der Transportvorrichtung 4 im zeitlichen Wechsel zwischen Stopp und einer Nenn-Transportgeschwindigkeit steuert.

Die Transportgeschwindigkeit 11 kann basierend auf Daten einer Erfassungsvorrichtung 12, beispielsweise einer Bilderfassungsvorrichtung, beispielsweise einer Kamera, gesteuert werden. Die Erfassungsvorrichtung 12 ist beispielhaft im Bereich eines Einlaufs 13 des Systems 1 angeordnet und zur Erfassung der Behälter 3 im Bereich des Einlaufs 13 des Systems 1 vorgesehen. Alternativ oder zusätzlich kann die/eine Erfassungsvorrichtung im Bereich eines Auslaufs 14 des Systems 1 angeordnet sein zur Erfassung der Behälter im Bereich des Auslaufs 14 des Systems 1. Im Folgenden wird auf diese letztgenannte Alternative nicht weiter eingegangen. Die Erfassungsvorrichtung 12 im Bereich des Einlaufs 13 des Systems 1 kann kontinuierlich Daten, beispielsweise Bilder der Behälter 3 im Bereich des Einlaufs 13 erfassen. Durch bekannte Methoden der Bildauswertung kann beispielsweise aus den Daten eine Anzahl an Behältern 3 in dem Einlauf 13 ermittelt werden.

Abhängig von der ermittelten Anzahl an Behältern 3 im Einlauf 13 kann die Steuerungsvorrichtung 10 die Transportgeschwindigkeit 11 der Transportvorrichtung 4 steuern. Dies umfasst beispielsweise, dass die Steuerungsvorrichtung 10 die Transportvorrichtung 4 stoppt, falls die Anzahl der Behälter 3 im Einlauf 13 eine vorgegebene Minimalanzahl unterschreitet und die Transportgeschwindigkeit 11 auf eine Nenn-Transportgeschwindigkeit steuert, falls die Anzahl der Behälter 3 im Einlauf 13 eine vorgegebene Maximalanzahl übersteigt. Die Minimalanzahl ist dabei geringer als die Maximalanzahl.

Außerdem kann das Temperaturprofil 7 durch die Steuerungsvorrichtung 10 gesteuert werden. Dabei umfasst eine Steuerung des Temperaturprofils 7 eine Steuerung der Temperaturen 7a-7g der Temperaturzonen 8a-8g. Das Temperaturprofil 7 wird dabei beispielsweise abhängig von einem prognostizierten Systemparameter 15, beispielsweise einer Transportgeschwindigkeit des Systems, gesteuert. Der Systemparameter 15 wird von der Steuerungsvorrichtung 10 beispielsweise basierend auf einer Transportgeschwindigkeit eines vorgeordneten Systems 16, beispielsweise einer Abfüllmaschine, prognostiziert. Alternativ kann die Steuerungsvorrichtung 10 den Systemparameter 15 auch basierend auf einer Transportgeschwindigkeit eines nachgeordneten Systems 17, beispielsweise einer Etikettiermaschine, prognostizieren. Im Folgenden wird auf diese letztgenannte Alternative nicht weiter eingegangen. Das vorgeordnete System 16 sendet eine Information über seine gegenwärtige Transportgeschwindigkeit an die Steuerungsvorrichtung. Hierbei kann die Steuerungsvorrichtung beispielsweise basierend auf dieser Information und der der Steuerungsvorrichtung 10 bekannten Zeit, die die Behälter brauchen, um von dem vorgeordneten System 16 zu dem System 1 zu gelangen, die Transportgeschwindigkeit des Systems in der Zukunft prognostizieren. Abhängig von der prognostizierten Transportgeschwindigkeit des Systems 1 kann die Steuerungsvorrichtung 10 dann beispielsweise das Temperaturprofil 7 einstellen bzw. ändern oder steuern. Die Steuerungsvorrichtung 10 kann z.B. die Temperaturen 7a-7g einzelner Temperaturzonen 8a-8g derart erhöhen, dass mehrere der Temperaturzonen 8a-8g die Maximaltemperatur aufweisen, bei der das Pasteurisieren der Behälter 3 stattfindet, falls die prognostizierte Transportgeschwindigkeit des Systems 1 gegenüber einem vorhergehenden Wert zunimmt. Andererseits kann die Steuerungsvorrichtung 10 die Temperaturen 7a-7g einzelner Temperaturzonen 8a-8g derart reduzieren, dass weniger der Temperaturzonen 8a-8g die Maximaltemperatur aufweisen, bei der das Pasteurisieren der Behälter 3 stattfindet, falls die prognostizierte Transportgeschwindigkeit des Systems 1 gegenüber einem vorhergehenden Wert abnimmt. Dabei weist jederzeit mindestens eine der Temperaturzonen 8a-8g eine zum Pasteurisieren der Behälter 3 benötigte Temperatur auf.

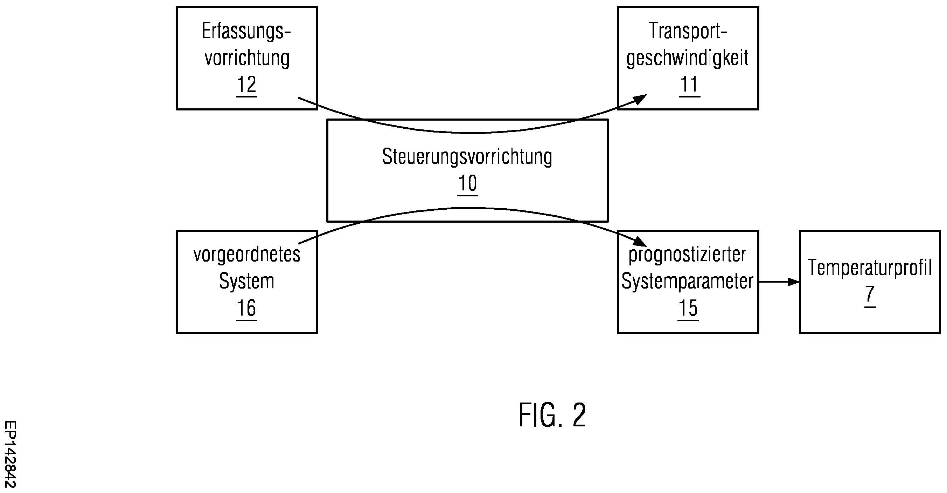

Fig. 2 zeigt den Ablauf von Vorgängen der Steuerungsvorrichtung 10 während des Betriebs des Systems 1 der Fig. 1 zum Erwärmen und/oder Abkühlen, insbesondere Pasteurisieren, von mit einem insbesondere flüssigen Füllgut gefüllten und geschlossenen Behältern in einem Blockdiagramm.

Zum einen erhält die Steuerungsvorrichtung 10 Daten, beispielsweise Bilder der Behälter 3 im Bereich des Einlaufs 13, von der Erfassungsvorrichtung 13, beispielsweise einer Bilderfassungsvorrichtung, beispielsweise einer Kamera. Durch bekannte Methoden der Bildauswertung kann die Steuerungsvorrichtung 10 z.B. aus den Daten eine Anzahl an Behältern 3 in dem Einlauf 13 ermitteln. Abhängig von der ermittelten Anzahl an Behältern 3 im Einlauf 13 kann die Steuerungsvorrichtung 10 die Transportgeschwindigkeit 11 der Transportvorrichtung 4 steuern. Dies umfasst, dass die Steuerungsvorrichtung 10 die Transportvorrichtung 4 stoppen kann, falls die Anzahl der Behälter 3 im Einlauf 13 eine vorgegebene Minimalanzahl unterschreitet und die Transportgeschwindigkeit 11 auf eine Nenn-Transportgeschwindigkeit steuern kann, falls die Anzahl der Behälter 3 im Einlauf 13 eine vorgegebene Maximalanzahl übersteigt. Die Minimalanzahl ist dabei geringer als die Maximalanzahl.

Zum anderen erhält die Steuerungsvorrichtung 10 eine Information über eine gegenwärtige Transportgeschwindigkeit des vorgeordneten Systems 16. Basierend auf dieser Information und der der Steuerungsvorrichtung 10 bekannten Zeit, die die Behälter 3 brauchen, um von dem vorgeordneten System 16 zu dem System 1 zu gelangen, kann die Steuerungsvorrichtung 10 den Systemparameter 15 prognostizieren, beispielsweise die Transportgeschwindigkeit des Systems in der Zukunft. Abhängig von dem prognostizierten Systemparameter 15 des Systems 1 steuert die Steuerungsvorrichtung 10 das Temperaturprofil 7 in der Zukunft.

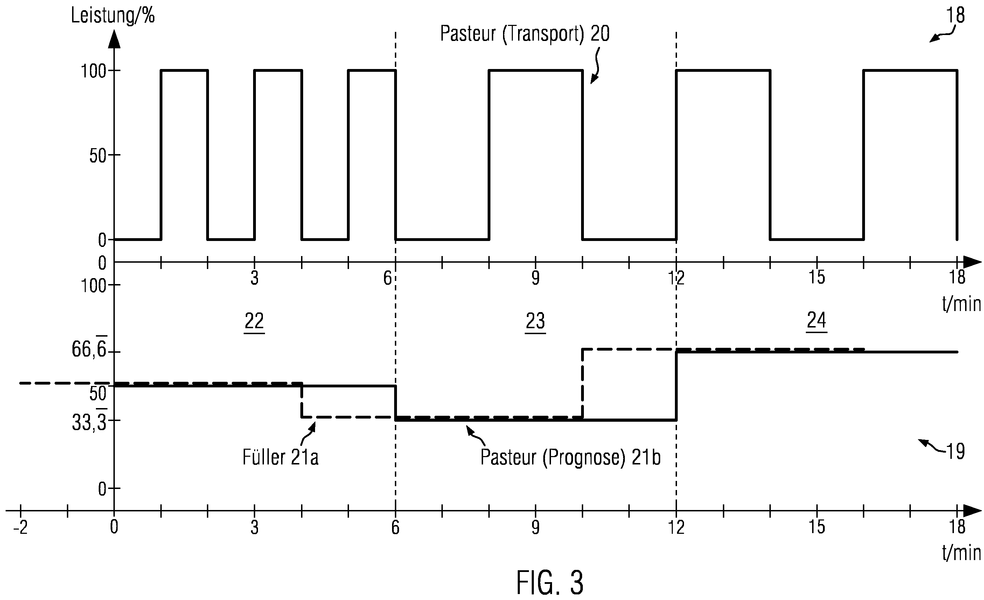

Fig. 3 zeigt schematisch einen beispielhaften zeitlichen Verlauf einer Transportleistung 20 eines Pasteurs, sowie eine prognostizierte Systemauslastung 21b des Pasteurs abhängig von der Leistung eines vorgeordneten Füllers 21a.

Dabei kann der zeitliche Verlauf der Transportleistung 20 einem zeitlichen Verlauf der Transportgeschwindigkeit 11 vorangegangener Ausführungsbeispiele entsprechen. Je höher/niedriger die Transportleistung 20 zu einem gegebenen Zeitpunkt, desto höher/niedriger ist die entsprechende Transportgeschwindigkeit 11. Der Pasteur kann ein Beispiel für ein System 1 sein, der vorgeordnete Füller kann ein Beispiel für ein vorgeordnetes System 16 aus vorangegangenen Ausführungsbeispielen sein. Die prognostizierte Systemauslastung 21b des Pasteurs kann dabei in dem prognostizierten Systemparameter vorangegangener Ausführungsformen umfasst sein. Entsprechend der prognostizierten Systemauslastung 21b des Pasteurs/ dem prognostizierten Systemparameter kann das Temperaturprofil 7 analog zu dem Ausführungsbeispiel der Fig. 1 angepasst werden. In diesem Beispiel kann die Leistung des vorgeordneten Füllers 21a der Transportgeschwindigkeit des Füllers entsprechen. Je höher/niedriger die Leistung des Füllers ist, desto höher/niedriger ist die entsprechende Transportgeschwindigkeit.

In einem unteren Teil 19 der Fig. 3 ist beispielhaft der zeitliche Verlauf der Leistung 21a des Füllers gezeigt. Von t=-2 min bis t=4 min, also in diesem Beispiel einer Zeitdauer von sechs Minuten, beträgt die Leistung des Füllers beispielsweise 50%. Nachfolgend, von t=4 min bis t=10 min kann der Füller unter einer Drittellast mit 33,3̅% Leistung laufen. Von t=10 min bis t=16 min kann die Leistung des Füllers zwei Drittel betragen, also 66,6̅%. In diesem Beispiel liegt der zeitliche Verlauf der Leistung des Füllers 21a zwei Minuten vor der prognostizierten Systemauslastung des Pasteurs 21b. Der zeitliche Verlauf des Füllers 21a kann als Grundlage für das Prognostizieren der Systemauslastung des Pasteurs 21b dienen. In dem gezeigten Beispiel kann der Verlauf der Leistung des Füllers 21a genau so weit zeitlich früher als der Verlauf der Systemauslastung des Pasteurs 21b liegen wie die Behälter 3 benötigen, um von dem Füller zu dem Pasteur transportiert zu werden, in diesem Beispiel zwei Minuten. Im vorliegenden Fall kann die Systemauslastung des Pasteurs 21b entsprechend der Leistung des Füllers 21a prognostiziert werden. Folglich wird die Systemauslastung des Pasteurs 21b in einem ersten Zeitabschnitt 22 von t=0 min bis t=6 min zu 50%, in einem zweiten Zeitabschnitt 23 von t=6 min bis t=12 min zu 33,3%, und in einem dritten Zeitabschnitt 24 von t=12 min bis t=18 min zu 66,6̅% prognostiziert. Entsprechend der prognostizierten Systemauslastung 21b des Pasteurs/ dem prognostizierten Systemparameter kann in diesem Beispiel das Temperaturprofil 7 analog zu dem Ausführungsbeispiel der Fig. 1 angepasst werden.

In einem oberen Teil 20 der Fig. 3 ist zur Veranschaulichung dieses Beispiels der zeitliche Verlauf der Transportleistung des Pasteurs 20 gezeigt. Die Transportleistung des Pasteurs 20, und damit der zeitliche Verlauf der Transportgeschwindigkeit 11, zeigt in diesem Beispiel über sämtliche Zeitabschnitte 22, 23, und 24 einen zeitlichen Wechsel zwischen Stopp (0% Leistung) und einer Nenn-Transportgeschwindigkeit (100% Leistung). Dabei kann die momentane Transportleistung/Transportgeschwindigkeit des Pasteurs abhängig von einer gegenwärtigen Anzahl an Behältern in einem Einlauf des Pasteurs analog zu dem Ausführungsbeispiel der Fig. 2 gesteuert werden. Über jeweils einen Zeitabschnitt 22, 23, oder 24 gemittelt kann die Transportleistung des Pasteurs 20 in dem gezeigten Beispiel über die Steuerung anhand der gegenwärtigen Anzahl an Behältern in dem Einlauf des Pasteurs mit einem Versatz von zwei Minuten der jeweiligen Leistung des vorgeordneten Füllers 21a entsprechen. Dadurch wird ein kontinuierlicher Betrieb erreicht. Einem langsamen Betrieb des Füllers entspricht ein Wechseltransportbetrieb des Pasteurs mit vermehrten Stoppzeiten und einem schnellen Betrieb des Füllers entspricht ein Wechseltransportbetrieb des Pasteurs mit verlängerten Nennlastzeiten.

### Bezugszeichenliste:

- 1: System
- 2: Füllgut
- 3: Behälter
- 4: Transportvorrichtung
- 5: Transportrichtung
- 6: Vorrichtung
- 6a: Behandlungsmedium
- 7: Temperaturprofil
- 7a-7g: Temperaturen
- 8a-8g: Temperaturzonen
- 9: Temperaturkurve
- 10: Steuerungsvorrichtung
- 11: Transportgeschwindigkeit
- 12: Erfassungsvorrichtung
- 13: Einlauf
- 14: Auslauf
- 15: prognostizierter Systemparameter
- 16: vorgeordnetes System
- 17: nachgeordnetes System
- 18: oberer Teil
- 19: unterer Teil
- 20: Transportleistung eines Pasteurs
- 21a: Leistung eines vorgeordneten Füllers
- 21b: prognostizierte Systemauslastung eines Pasteurs
- 22: erster Zeitabschnitt
- 23: zweiter Zeitabschnitt
- 24: dritter Zeitabschnitt

## Patentansprüche

1. System (1) zum Erwärmen und/oder Abkühlen, insbesondere Pasteurisieren, von mit einem insbesondere flüssigen Füllgut (2) gefüllten und geschlossenen Behältern (3) umfassend:
eine Transportvorrichtung (4) ausgebildet zum Transportieren der Behälter in einer Transportrichtung (5),
eine Vorrichtung (6) ausgebildet zum Erwärmen und/oder Abkühlen der Behälter gemäß einem Temperaturprofil (7) entlang der Transportrichtung, und
eine Steuerungsvorrichtung (10) ausgebildet zum Steuern einer Transportgeschwindigkeit (11) der Transportvorrichtung und des Temperaturprofils,
wobei die Steuerungsvorrichtung ausgebildet ist, die Transportgeschwindigkeit im zeitlichen Wechsel zwischen Stopp und einer Nenn-Transportgeschwindigkeit zu steuern,
wobei die Steuerungsvorrichtung ausgebildet ist, das Temperaturprofil abhängig von einem prognostizierten Systemparameter (15) zu steuern,
wobei die Steuerungsvorrichtung ausgebildet ist die Transportgeschwindigkeit abhängig von:
- einer Anzahl an Behältern in einem Einlauf (13) und/oder Auslauf (14) des Systems in der Vergangenheit und/oder Gegenwart, insbesondere deren zeitlicher Verläufe und/oder
- einer verfügbaren Kapazität zur Aufnahme von Behältern in dem/einem Einlauf und/oder Auslauf des Systems in der Vergangenheit und/oder Gegenwart, insbesondere deren zeitlicher Verläufe, zu steuern.

2. System nach Anspruch 1, wobei der Systemparameter die Transportgeschwindigkeit, eine Anzahl der in einer Zeiteinheit einlaufenden Behälter, eine Anzahl der in einer Zeiteinheit abgebbaren Behälter, einer Länge des vollen Teils einer Staustrecke, einer Anzahl von (gestauten) Behältern in einer Staustrecke, eine Aufnahme- und/oder Ausgabekapazität, und/oder eine Systemauslastung in Prozent, jeweils des Systems, umfasst.

3. System nach einem der vorhergehenden Ansprüche umfassend eine Erfassungsvorrichtung (12) im Bereich des/eines Einlaufs und/oder Auslaufs des Systems zur Erfassung von Behältern im Bereich des Einlaufs und/oder Auslaufs des Systems,
wobei die Erfassungsvorrichtung einen Stauschalter und/oder eine Bilderfassungsvorrichtung umfasst,
insbesondere wobei die Steuerungsvorrichtung ausgebildet ist die Transportgeschwindigkeit basierend auf Daten der Erfassungsvorrichtung zu steuern.

4. System nach einem der vorhergehenden Ansprüche, wobei die Steuerungsvorrichtung ausgebildet ist den Systemparameter basierend auf einer Transportgeschwindigkeit, einer Anzahl der in einer Zeiteinheit einlaufenden Behälter, einer Anzahl der in einer Zeiteinheit abgebbaren Behälter, der/einer Länge des vollen Teils einer Staustrecke, einer Anzahl von (gestauten) Behältern in einer Staustrecke, einer Aufnahme- und/oder Ausgabekapazität, und/oder einer Systemauslastung in Prozent jeweils des Systems, und/oder eines oder mehrerer dem System vor- (16) und/oder nachgeordneten (17) weiteren Systemen in der Vergangenheit und/oder Gegenwart zu prognostizieren.

5. System nach einem der vorhergehenden Ansprüche, wobei die Vorrichtung entlang der Transportrichtung der Behälter mehrere Temperaturzonen (8a-g) aufweist,
wobei das Temperaturprofil steuerbare Temperaturen (7a-g) der Temperaturzonen umfasst und eine Steuerung des Temperaturprofils eine Steuerung der Temperaturen der Temperaturzonen umfasst,
wobei jede Temperaturzone eine für sich genommen einheitliche Temperatur aufweist.

6. System nach Anspruch 5, wobei in mehreren als Aufwärmzonen bezeichneten Temperaturzonen, die in der Transportrichtung aufeinanderfolgen, die Temperaturen von Temperaturzone zu Temperaturzone ansteigen oder gleichbleiben und
wobei in wenigstens einer oder mehreren in der Transportrichtung den Aufwärmzonen nachfolgenden als Pasteurisierungszonen bezeichneten Temperaturzonen die Temperatur einer Maximaltemperatur entspricht und
wobei in mehreren in der Transportrichtung den Pasteurisierungszonen nachfolgenden als Abkühlzonen bezeichneten Temperaturzonen die Temperaturen von Temperaturzone zu Temperaturzone abnehmen oder gleichbleiben.

7. System nach Anspruch 6, wobei die Steuerungsvorrichtung ausgebildet ist:
- eine Anzahl der Pasteurisierungszonen zu erhöhen, falls
a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, zunimmt und/oder
b) eine prognostizierte Anzahl der in einer Zeiteinheit abgebbaren Behälter, und/oder eine prognostizierte Aufnahmekapazität, jeweils des Systems, abnimmt
und/oder
- eine/die Anzahl der Pasteurisierungszonen zu reduzieren, falls
a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, abnimmt und/oder
b) eine prognostizierte Anzahl der in einer Zeiteinheit abgebbaren Behälter, und/oder eine prognostizierte Aufnahmekapazität, jeweils des Systems, zunimmt.

8. System nach Anspruch 7, wobei die Steuerungsvorrichtung ausgebildet ist die Transportgeschwindigkeit, die Anzahl der in einer Zeiteinheit einlaufenden Behälter, die Ausgabekapazität, die Anzahl der in einer Zeiteinheit abgebbaren Behälter, die Aufnahmekapazität, und/oder die Systemauslastung in Prozent basierend auf der/einer Transportgeschwindigkeit, der/einer Anzahl der in einer Zeiteinheit einlaufenden Behälter, der/einer Anzahl der in einer Zeiteinheit abgebbaren Behälter, der/einer Länge des vollen Teils einer Staustrecke, einer Anzahl von (gestauten) Behältern in einer Staustrecke, der/einer Aufnahme- und/oder Ausgabekapazität, und/oder der/einer Systemauslastung in Prozent jeweils des Systems, und/oder eines oder mehrerer dem System vor- und/oder nachgeordneten weiteren Systemen in der Vergangenheit und/oder Gegenwart zu prognostizieren.

9. Verfahren zum Erwärmen und/oder Abkühlen, insbesondere Pasteurisieren, von mit einem insbesondere flüssigen Füllgut (2) gefüllten und verschlossenen Behältern (3) mithilfe eines Systems (1) zum Erwärmen und/oder Abkühlen, insbesondere Pasteurisieren, von den verschlossenen Behältern (3) umfassend:
eine Transportvorrichtung (4) ausgebildet zum Transportieren der Behälter in einer Transportrichtung (5),
eine Vorrichtung (6) ausgebildet zum Erwärmen und/oder Abkühlen der Behälter gemäß einem Temperaturprofil (7) entlang der Transportrichtung, und
eine Steuerungsvorrichtung (10) ausgebildet zum Steuern einer Transportgeschwindigkeit (11) der Transportvorrichtung und des Temperaturprofils,
wobei die Steuerungsvorrichtung ausgebildet ist, die Transportgeschwindigkeit im zeitlichen Wechsel zwischen Stopp und einer Nenn-Transportgeschwindigkeit zu steuern,
wobei die Steuerungsvorrichtung ausgebildet ist, das Temperaturprofil abhängig von einem prognostizierten Systemparameter (15) zu steuern,
wobei das Verfahren die folgenden Schritte umfasst:
Transportieren der Behälter in der Transportrichtung (5),
Erwärmen und/oder Abkühlen der Behälter gemäß dem Temperaturprofil (7) entlang der Transportrichtung,
Steuern der Transportgeschwindigkeit (11) der Transportvorrichtung (4) und des Temperaturprofils, wobei die Transportgeschwindigkeit im zeitlichen Verlauf zwischen Stopp und der Nenn-Transportgeschwindigkeit wechselt,
Prognostizieren des Systemparameters (15), und
Steuern des Temperaturprofils abhängig von dem prognostizierten Systemparameter.

10. Verfahren nach Anspruch 9, wobei die Transportgeschwindigkeit abhängig von:
- einer Anzahl an Behältern in dem/einem Einlauf (13) und/oder Auslauf (14) des Systems in der Vergangenheit und/oder Gegenwart, insbesondere deren zeitlicher Verläufe und/oder
- einer verfügbaren Kapazität zur Aufnahme von Behältern in dem/einem Einlauf und/oder Auslauf des Systems in der Vergangenheit und/oder Gegenwart, insbesondere deren zeitlicher Verläufe, gesteuert wird.

11. Verfahren nach Anspruch 9 oder 10 umfassend Erfassen von Behältern im Bereich des/eines Einlaufs und/oder Auslaufs des Systems,
wobei das Erfassen ein Erfassen eines Staus und/oder eines Bildes umfasst,
insbesondere wobei die Transportgeschwindigkeit basierend auf Daten des Erfassens gesteuert wird.

12. Verfahren nach einem der Ansprüche 9 bis 11 umfassend Prognostizieren des Systemparameters basierend auf einer Transportgeschwindigkeit, einer Anzahl der in einer Zeiteinheit einlaufenden Behälter, einer Anzahl der in einer Zeiteinheit abgebbaren Behälter, der/einer Länge des vollen Teils einer Staustrecke, einer Anzahl von (gestauten) Behältern in einer Staustrecke, einer Aufnahme- und/oder Ausgabekapazität, und/oder einer Systemauslastung in Prozent jeweils des Systems, und/oder eines oder mehrerer dem System vor- (16) und/oder nachgeordneten (17) weiteren Systemen in der Vergangenheit und/oder Gegenwart.

13. Verfahren nach einem der Ansprüche 9 bis 12 bezogen auf ein System des Anspruchs 7 oder 8 umfassend:
- Erhöhen einer Anzahl der Pasteurisierungszonen, falls
a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, zunimmt und/oder
b) eine prognostizierte Anzahl der in einer Zeiteinheit abgebbaren Behälter, und/oder eine prognostizierte Aufnahmekapazität, jeweils des Systems, abnimmt
und/oder
- Reduzieren einer/der Anzahl der Pasteurisierungszonen, falls
a) eine prognostizierte Transportgeschwindigkeit, eine prognostizierte Anzahl der in einer Zeiteinheit einlaufenden Behälter, eine prognostizierte Ausgabekapazität, und/oder eine prognostizierte Systemauslastung in Prozent, jeweils des Systems, abnimmt und/oder
b) eine prognostizierte Anzahl der in einer Zeiteinheit abgebbaren Behälter, und/oder eine prognostizierte Aufnahmekapazität, jeweils des Systems, zunimmt.

14. Verfahren nach Anspruch 13, umfassend Prognostizieren der Transportgeschwindigkeit, der Anzahl der in einer Zeiteinheit einlaufenden Behälter, der Ausgabekapazität, der Anzahl der in einer Zeiteinheit abgebbaren Behälter, der Aufnahmekapazität, und/oder der Systemauslastung in Prozent basierend auf der/einer Transportgeschwindigkeit, der/einer Anzahl der in einer Zeiteinheit einlaufenden Behälter, der/einer Anzahl der in einer Zeiteinheit abgebbaren Behälter, der/einer Länge des vollen Teils einer Staustrecke, einer Anzahl von (gestauten) Behältern in einer Staustrecke, der/einer Aufnahme- und/oder Ausgabekapazität, und/oder der/einer Systemauslastung in Prozent jeweils des Systems, und/oder eines oder mehrerer dem System vor- und/oder nachgeordneten weiteren Systemen in der Vergangenheit und/oder Gegenwart.

## Claims

1. System (1) for heating and/or cooling, in particular pasteurizing, containers (3) filled with a particularly liquid product (2) and closed, comprising:
a transport device (4) designed to transport the containers in a transport direction (5),
a device (6) designed to heat and/or cool the containers according to a temperature profile (7) along the transport direction, and
a control device (10) designed to control a transport speed (11) of the transport device and the temperature profile,
wherein the control device is designed to control the transport speed in a time-varying manner between stop and a nominal transport speed,
wherein the control device is designed to control the temperature profile as a function of a predicted system parameter (15),
wherein the control device is designed to control the transport speed as a function of:
- a number of containers in an inlet (13) and/or outlet (14) of the system in the past and/or present, in particular their temporal sequences, and/or
- an available capacity for receiving containers in the inlet and/or outlet of the system in the past and/or present, in particular their temporal sequences.

2. System according to claim 1, wherein the system parameter comprises the transport speed, a number of containers arriving in a time unit, a number of containers that can be delivered in a time unit, a length of the full part of a congestion section, a number of (congested) containers in a congestion section, a receiving and/or delivery capacity, and/or a system utilization in percent, in each case of the system.

3. System according to one of the preceding claims, comprising a detection device (12) in the area of the inflow and/or outflow of the system for detecting containers in the area of the inflow and/or outflow of the system,
wherein the detection device comprises a jam switch and/or an image detection device,
in particular wherein the control device is designed to control the transport speed based on data from the detection device.

4. System according to one of the preceding claims, wherein the control device is designed to control the system parameters based on a transport speed, a number of containers arriving in a time unit, a number of containers that can be delivered in a time unit, the length of the full part of a jam section, a number of (congested) containers in a congestion section, a receiving and/or output capacity, and/or a system utilization in percent of the system, and/or one or more further systems upstream (16) and/or downstream (17) of the system in the past and/or present.

5. System according to one of the preceding claims, wherein the device has several temperature zones (8a-g) along the transport direction of the containers,
wherein the temperature profile comprises controllable temperatures (7a-g) of the temperature zones and control of the temperature profile comprises control of the temperatures of the temperature zones,
wherein each temperature zone has a uniform temperature in itself.

6. System according to claim 5, wherein in a plurality of temperature zones referred to as warming zones, which follow one another in the transport direction, the temperatures rise or remain the same from temperature zone to temperature zone, and
wherein in at least one or more temperature zones referred to as pasteurization zones, which follow the warming zones in the transport direction, the temperature corresponds to a maximum temperature, and
wherein, in a plurality of temperature zones designated as cooling zones and following the pasteurization zones in the transport direction, the temperatures decrease or remain constant from temperature zone to temperature zone.

7. System according to claim 6, wherein the control device is designed:
- increase the number of pasteurization zones if
a) a predicted transport speed, a predicted number of containers arriving in a unit of time, a predicted output capacity, and/or a predicted system utilization in percent, each of the system, increases and/or
b) a predicted number of containers that can be discharged in a time unit and/or a predicted intake capacity, each of the system, decreases
and/or
- reduce the number of pasteurization zones if
a) a predicted transport speed, a predicted number of containers arriving in a unit of time, a predicted output capacity, and/or a predicted system utilization in percent, each of the system, decreases and/or
b) a predicted number of containers that can be discharged in a unit of time and/or a predicted intake capacity, in each case of the system, increases.

8. System according to claim 7, wherein the control device is designed to determine the transport speed, the number of containers arriving in a time unit, the output capacity, the number of containers that can be delivered in a time unit, the intake capacity, and/or the system utilization in percent based on the transport speed, the number of containers arriving in a time unit, the number of containers discharged in a time unit, the length of the full part of a congestion section, a number of (congested) containers in a congestion section, the intake and/or output capacity, and/or the system utilization in percent, in each case of the system, and/or one or more further systems upstream and/or downstream of the system in the past and/or present.

9. Method for heating and/or cooling, in particular pasteurizing, containers (3) filled with a particularly liquid filling (2) and sealed, using a system (1) for heating and/or cooling, in particular pasteurizing, the sealed containers (3), comprising:
a transport device (4) designed to transport the containers in a transport direction (5),
a device (6) designed to heat and/or cool the containers according to a temperature profile (7) along the transport direction, and
a control device (10) designed to control a transport speed (11) of the transport device and the temperature profile,
wherein the control device is designed to control the transport speed in alternation between stop and a nominal transport speed,
wherein the control device is designed to control the temperature profile as a function of a predicted system parameter (15),
wherein the method comprises the following steps:
transporting the containers in the transport direction (5),
heating and/or cooling the containers according to the temperature profile (7) along the transport direction,
controlling the transport speed (11) of the transport device (4) and the temperature profile, wherein the transport speed alternates between stop and the nominal transport speed over time,
predicting the system parameter (15), and
controlling the temperature profile depending on the predicted system parameter.

10. Method according to claim 9, wherein the transport speed is controlled depending on:
- a number of containers in the inlet (13) and/or outlet (14) of the system in the past and/or present, in particular their temporal courses, and/or
- an available capacity for receiving containers in the inlet and/or outlet of the system in the past and/or present, in particular their temporal courses.

11. Method according to claim 9 or 10, comprising detecting containers in the area of the inlet and/or outlet of the system,
wherein the detection comprises detecting a jam and/or an image,
in particular wherein the transport speed is controlled based on data from the detection.

12. Method according to one of claims 9 to 11, comprising predicting the system parameter based on a transport speed, a number of containers arriving in a time unit, a number of containers that can be delivered in a time unit, the length of the full part of a congestion section, a number of (congested) containers in a congestion section, a receiving and/or output capacity, and/or a system utilization in percent, in each case of the system, and/or one or more further systems upstream (16) and/or downstream (17) of the system in the past and/or present.

13. Method according to one of claims 9 to 12 relating to a system according to claim 7 or 8, comprising:
- increasing a number of pasteurization zones if
a) a predicted transport speed, a predicted number of containers arriving in a time unit, a predicted output capacity, and/or a predicted system utilization in percent, each of the system, increases and/or
b) a predicted number of containers that can be discharged in a unit of time, and/or a predicted intake capacity, each of the system, decreases
and/or
- Reducing the number of pasteurization zones if
a) a predicted transport speed, a predicted number of containers arriving in a unit of time, a predicted output capacity, and/or a predicted system utilization in percent, each of the system, decreases and/or
b) a predicted number of containers that can be discharged in a unit of time and/or a predicted intake capacity, each of the system, increases.

14. Method according to claim 13, comprising predicting the transport speed, the number of containers arriving in a time unit, the output capacity, the number of containers that can be delivered in a time unit, the intake capacity, and/or the system utilization in percent based on the transport speed, the number of containers arriving in a time unit, the number of containers that can be delivered in a time unit, the length of the full part of a congestion section, a number of (congested) containers in a congestion section, the intake and/or output capacity, and/or the system utilization in percent, in each case of the system, and/or one or more further systems upstream and/or downstream of the system in the past and/or present.

## Revendications

1. Système (1) de chauffage et/ou de refroidissement, en particulier de pasteurisation, de récipients (3) fermés et remplis d'un produit de remplissage (2), en particulier liquide, comprenant :
un dispositif de transport (4) conçu pour transporter les récipients dans une direction de transport (5),
un dispositif (6) conçu pour chauffer et/ou refroidir les récipients selon un profil de température (7) le long de la direction de transport, et
un dispositif de contrôle (10) conçu pour contrôler une vitesse de transport (11) du dispositif de transport et le profil de température,
dans lequel le dispositif de contrôle est conçu pour contrôler la vitesse de transport en alternance temporelle entre l'arrêt et une vitesse de transport nominale,
dans lequel le dispositif de contrôle est conçu pour contrôler le profil de température en fonction d'un paramètre de système (15) prédit,
dans lequel le dispositif de contrôle est conçu pour contrôler la vitesse de transport en fonction :
- d'un nombre de récipients à une entrée (13) et/ou à une sortie (14) du système dans le passé et/ou actuellement, en particulier de son évolution temporelle, et/ou
- d'une capacité disponible d'admission de récipients à la ou à une entrée et/ou à la ou à une sortie du système dans le passé et/ou actuellement, en particulier de son évolution temporelle.

2. Système selon la revendication 1, dans lequel le paramètre de système comprend la vitesse de transport, un nombre de récipients entrant par unité de temps, un nombre de récipients traitables par unité de temps, une longueur de la section pleine d'une ligne d'accumulation, un nombre de récipients (accumulés) dans une ligne d'accumulation, une capacité d'admission et/ou de traitement, et/ou un taux d'utilisation en pourcentage respectifs du système.

3. Système selon l'une des revendications précédentes, comprenant un dispositif de détection (12) dans la zone de la ou d'une entrée et/ou de la ou d'une sortie du système pour détecter des récipients dans la zone d'entrée et/ou de sortie du système,
dans lequel le dispositif de détection comprend un commutateur d'accumulation et/ou un dispositif de capture d'image,
en particulier dans lequel le dispositif de contrôle est conçu pour contrôler la vitesse de transport sur la base de données provenant du dispositif de détection.

4. Système selon l'une des revendications précédentes, dans lequel le dispositif de contrôle est conçu pour prédire le paramètre du système sur la base d'une vitesse de transport, d'un nombre de récipients entrant par unité de temps, d'un nombre de récipients traitables par unité de temps, de la ou d'une longueur de la section pleine d'une ligne d'accumulation, d'un nombre de récipients (accumulés) dans une ligne d'accumulation, d'une capacité d'admission et/ou de traitement et/ou d'un taux d'utilisation en pourcentage respectifs du système, et/ou d'un ou plusieurs autres systèmes en amont (16) et/ou en aval (17) du système dans le passé et/ou actuellement.

5. Système selon l'une des revendications précédentes, dans lequel le dispositif comporte plusieurs zones de température (8a-g) le long de la direction de transport des récipients,
dans lequel le profil de température comprend des températures contrôlables (7a-g) des zones de température, et un contrôle du profil de température comprend un contrôle des températures des zones de température,
dans lequel chaque zone de température présente une température uniforme correspondante.

6. Système selon la revendication 5, dans lequel, dans plusieurs zones de température désignées comme zones de préchauffage, qui se succèdent dans la direction de transport, les températures augmentent ou se maintiennent d'une zone de température à l'autre, et
dans lequel, dans au moins une zone de température désignée comme une zone de pasteurisation, qui succède aux zones de préchauffage dans la direction de transport, la température correspond à une température maximale, et
dans lequel, dans plusieurs zones de température désignées comme zones de refroidissement, qui succèdent aux zones de pasteurisation dans la direction de transport, les températures diminuent ou se maintiennent d'une zone de température à l'autre.

7. Système selon la revendication 6, dans lequel le dispositif de contrôle est conçu :
- pour augmenter le nombre de zones de pasteurisation dans le cas
a) d'une augmentation d'une vitesse de transport prédite, d'un nombre prédit de récipients entrant par unité de temps, d'une capacité de traitement prédite et/ou d'un taux d'utilisation prédit en pourcentage respectifs du système, et/ou
b) d'une diminution d'un nombre prédit de récipients traitables par unité de temps et/ou d'une capacité d'admission prédite respectifs du système,
et/ou
- pour réduire le ou un nombre de zones de pasteurisation dans le cas
a) d'une diminution d'une vitesse de transport prédite, d'un nombre prédit de récipients entrant par unité de temps, d'une capacité de traitement prédite et/ou d'un taux d'utilisation prédit en pourcentage respectifs du système, et/ou
b) d'une augmentation d'un nombre prédit de récipients traitables par unité de temps et/ou d'une capacité d'admission prédite respectifs du système.

8. Système selon la revendication 7, dans lequel le dispositif de contrôle est conçu pour prédire la vitesse de transport, le nombre de récipients entrant par unité de temps, la capacité de traitement, le nombre de récipients traitables par unité de temps, la capacité d'admission et/ou le taux d'utilisation du système en pourcentage, sur la base de la ou d'une vitesse de transport, du ou d'un nombre de récipients entrant par unité de temps, du ou d'un nombre de récipients traitables par unité de temps, de la ou d'une longueur de la section pleine d'une ligne d'accumulation, d'un nombre de récipients (accumulés) dans une ligne d'accumulation, de la ou d'une capacité d'admission et/ou de traitement, et/ou du ou d'un taux d'utilisation en pourcentage respectifs du système, et/ou d'un ou plusieurs autres systèmes en amont et/ou en aval du système dans le passé et/ou actuellement.

9. Procédé de chauffage et/ou de refroidissement, en particulier de pasteurisation, de récipients (3) remplis d'un produit de remplissage (2), en particulier liquide, et scellés, au moyen d'un système (1) de chauffage et/ou de refroidissement, en particulier de pasteurisation, des récipients (3) scellés, comprenant :
un dispositif de transport (4) conçu pour transporter les récipients dans une direction de transport (5),
un dispositif (6) conçu pour chauffer et/ou refroidir les récipients selon un profil de température (7) le long de la direction de transport, et
un dispositif de contrôle (10) conçu pour contrôler une vitesse de transport (11) du dispositif de transport et le profil de température,
dans lequel le dispositif de contrôle est conçu pour contrôler la vitesse de transport en alternance temporelle entre l'arrêt et une vitesse de transport nominale,
dans lequel le dispositif de contrôle est conçu pour contrôler le profil de température en fonction d'un paramètre de système (15) prédit,
dans lequel le procédé comprend les étapes suivantes :
transport des récipients dans la direction de transport (5),
chauffage et/ou refroidissement des récipients selon le profil de température (7) le long de la direction de transport,
contrôle de la vitesse de transport (11) du dispositif de transport (4) et du profil de température, dans lequel la vitesse de transport varie au fil du temps entre l'arrêt et la vitesse de transport nominale,
prédiction du paramètre de système (15), et
contrôle du profil de température en fonction du paramètre de système prédit.

10. Procédé selon la revendication 9, dans lequel la vitesse de transport est contrôlée en fonction :
- d'un nombre de récipients dans la ou une entrée (13) et/ou la ou une sortie (14) du système dans le passé et/ou actuellement, en particulier de son évolution temporelle, et/ou
- d'une capacité disponible d'admission de récipients à la ou à une entrée et/ou à la ou à une sortie du système dans le passé et/ou actuellement, en particulier de son évolution temporelle.

11. Procédé selon la revendication 9 ou 10, comprenant la détection de récipients dans la zone de la ou d'une entrée et/ou de la ou d'une sortie du système,
dans lequel la détection comprend la détection d'une accumulation et/ou la capture d'une image,
en particulier dans lequel la vitesse de transport est contrôlée sur la base des données de détection.

12. Procédé selon l'une des revendications 9 à 11, comprenant la prédiction du paramètre de système sur la base d'une vitesse de transport, d'un nombre de récipients entrant par unité de temps, d'un nombre de récipients traitables par unité de temps, de la ou d'une longueur de la section pleine d'une ligne d'accumulation, d'un nombre de récipients (accumulés) dans une ligne d'accumulation, d'une capacité d'admission et/ou de traitement et/ou d'un taux d'utilisation en pourcentage respectifs du système, et/ou d'un ou plusieurs autres systèmes en amont (16) et/ou en aval (17) du système dans le passé et/ou actuellement.

13. Procédé selon l'une des revendications 9 à 12 relatif à un système selon la revendication 7 ou 8, comprenant :
- l'augmentation du nombre de zones de pasteurisation, dans le cas
a) d'une augmentation d'une vitesse de transport prédite, d'un nombre prédit de récipients entrant par unité de temps, d'une capacité de traitement prédite et/ou d'un taux d'utilisation prédit en pourcentage respectifs du système, et/ou
b) d'une diminution d'un nombre prédit de récipients traitables par unité de temps et/ou d'une capacité d'admission prédite respectifs du système,
et/ou
- la réduction du ou d'un nombre de zones de pasteurisation, dans le cas
a) d'une diminution d'une vitesse de transport prédite, d'un nombre prédit de récipients entrant par unité de temps, d'une capacité de traitement prédite et/ou d'un taux d'utilisation prédit en pourcentage respectifs du système, et/ou
b) d'une augmentation d'un nombre prédit de récipients traitables par unité de temps et/ou d'une capacité d'admission prédite respectifs du système.

14. Procédé selon la revendication 13, comprenant la prédiction de la vitesse de transport, du nombre de récipients entrant par unité de temps, de la capacité de traitement, du nombre de récipients traitables par unité de temps, de la capacité d'admission et/ou du taux d'utilisation du système en pourcentage, sur la base de la ou d'une vitesse de transport, du ou d'un nombre de récipients entrant par unité de temps, du ou d'un nombre de récipients traitables par unité de temps, de la ou d'une longueur de la section pleine d'une ligne d'accumulation, d'un nombre de récipients (accumulés) dans une ligne d'accumulation, de la ou d'une capacité d'admission et/ou de traitement et/ou du ou d'un taux d'utilisation en pourcentage respectifs du système, et/ou d'un ou plusieurs autres systèmes en amont et/ou en aval du système dans le passé et/ou actuellement.
